# EUROPÄISCHE PATENTSCHRIFT

(11) **EP 2 704 753 B1**
(45) Veröffentlichungstag und Bekanntmachung des Hinweises auf die Patenterteilung: **13.07.2016**
(21) Anmeldenummer: 12718600.5
(22) Anmeldetag: 27.04.2012
(51) Int. Cl.: A61L 9/012, A61L 9/05, A61L 9/04, C11D 3/50, C11D 17/00

(54) **VERWENDUNG EINES STÜCKFÖRMIGEN SANITÄRMITTELS**
USE OF A SANITARY PRODUCT IN PIECE FORM
UTILISATION D'UN PRODUIT SANITAIRE EN FORME DE PIÈCE

(30) Priorität: 06.05.2011 DE 102011100859
(43) Veröffentlichungstag der Anmeldung: 12.03.2014
(73) Patentinhaber: Buck-Chemie GmbH, 71083 Herrenberg (DE)
(72) Erfinder: LEIPOLD, Joachim, 72760 Reutlingen (DE); FRITZ, Matthias, 72810 Gomaringen (DE); JAESCHKE, Edgar, 70794 Filderstadt (DE)
(74) Vertreter: Mammel und Maser
(86) Internationale Anmeldenummer: PCT/EP2012/001818
(87) Internationale Veröffentlichungsnummer: WO 2012/152388

(56) Entgegenhaltungen:
- EP-A1- 0 057 839
- EP-A1- 0 864 637
- WO-A1-2004/083280
- WO-A1-2009/100962
- DE-A1-102008 051 173

## Beschreibung

Die vorliegende Erfindung betrifft ein stückförmiges Mittel zur Beduftung von Toiletten, das für die Anwendung in einem Wasserkasten vorgesehen ist.

Toilettenreinigungsmittel, die gleichzeitig als Raumlufterfrischungsmittel durch Beduftung des Toilettenbereiches dienen, sind im Stand der Technik bekannt. Diese werden in der Regel an einem Halter oder in einem Korb oder käfigartigen Behälter im Toilettenbecken an einer Stelle angebracht, die bei jedem Spülvorgang von zulaufendem Spülwasser durchströmt wird, wodurch sich das Mittel nach und nach verbraucht.

Die Lebensdauer dieser Toilettenreinigungsmittel wird daher durch die Anzahl der Spülungen in den Toiletten, in deren Toilettenbecken sie angebracht sind, bestimmt. Diese erzielte Lebensdauer und der Gehalt an Duftstoffen für die Raumbeduftung sind dabei regelmäßig an dem Gebrauch durch eine 3-bis 4-köpfige Familie orientiert, so dass eine ausreichende Raumbeduftung dann nicht mehr stattfinden kann, wenn der Haushalt oder der Ort, in deren Bereich die das Reinigungsmittel enthaltende Toilette sich befindet, von weniger Personen und/oder nicht dauerhaft benutzt wird. Gleichzeitig sorgen die in solchen Reinigungsmitteln enthaltenen Abspülregulative in Form z. B. nichtionischer Tenside dafür, dass die Duftstoffe mehr oder weniger zurückgehalten werden und nicht in die Umgebung des Toilettenbereiches verdampfen können.

Die DE 197 10 635 A1 lehrt ein Mittel, das zur Befestigung am Toilettenrand dient und das eine ausreichende und permanente Raumbeduftung auch bei einer geringen Anzahl von Spülzyklen erreicht. Das Mittel umfasst Duftstoffe und liegt als Lyogel, also als ein an Flüssigkeit reiches disperses System aus mindestens zwei Komponenten, nämlich einem festen, kolloid zerteilten Gelbildner und einer Flüssigkeit als Dispersionsmittel, vor. Diese Mittel lösen sich beim Überspülen mit Wasser in einem Körbchen im Allgemeinen nach 100 bis 250 Spülungen auf und entsprechen infolge ihrer Transparenz auch den Verbraucherwünschen.

Aus dem US-Patent 4,666,671 sind Raumerfrischungsblocks für den Toilettenbereich bekannt, die Gelbildner, Duftstoffe sowie Lösungsmittel enthalten. Mit diesen in Gelform vorliegenden Raumerfrischungsblocks kann zwar auch bei wenig oder nicht dauerhaft benutzten Toiletten eine konstante Raumbeduftung erreicht werden. Diese Blocks dienen jedoch ausschließlich der Raumerfrischung des Toilettenbereichs und lösen sich nicht auf.

Aus der EP 1 553 162 B1 sind Mittel mit einer festen Phase und einer transparenten Gelphase auf Polyamidharzbasis mit Duftstoffen zur Beduftung bekannt, die zur Anwendung in einem WC-Körbchen dienen. Nachteilig ist an diesen Mitteln jedoch, dass sich die Gelphase im Toilettenkörbchen beim Überspülen mit Wasser nicht auflöst sondern als Rückstand entsorgt werden muss.

Aus der EP 1 632 251 A1 ist ein Beduftungsprodukt für den Sanitärbereich bekannt, das aus zwei getrennten festen Trägern besteht. Einer der Träger ist transparent, die Mittel sind - um ein ansprechendes Produkt zu erhalten - vorzugsweise zweifarbig.

Die WO 2009/10962 A1 lehrt Wasch- und Reinigungsmittel mit porösen Polyamid-Partikeln. Der Anteil an Polyamiden in diesem Mittel liegt bei maximal 10 %.

Aus der DE 10 2008 051 173 A1 sind haftende Mittel zur Applikation auf einem Sanitärgegenstand bekannt.

Die EP 0 057 839 A1 lehrt Gelmassen auf Basis einer Polyurethan-Matrix und höher molekularen Polyolen.

Aus der WO 2004/083280 A1 und der US 2004/186263 A1 sind Polyamidpolyetherblockcopolymere bekannt.

Für die Anwendung im Wasserkasten sind Reinigungs- und/oder Beduftungsmittel bekannt, die Tenside, Duftstoffe, teilweise auch Farbstoffe, Bleichmittel etc. umfassen und extrudiert werden. Diese Mittel dienen zur Lagerung in kaltem Wasser, lösen sich im Allgemeinen erst nach 400 oder mehr Spülungen vollständig auf und sind nicht transparent.

Weiterhin sind zweiphasige Mittel für den Wasserkasten bekannt, beispielsweise der "Blink Blauspüter" der Firma Budich International, Hiddenhausen, der neben einem reinigenden Blauspüler als zweite Phase einen Kalklösekern umfasst oder der "Bloo Power Core" der Firma Jeyes, der neben einer blauspülenden Reinigungsmittelformkörperphase als zweite Phase einen sich schnell lösenden Teil mit weiteren aktiven Phasen mit Duft und Schaum umfasst.

Die Beduftung der Toilette mit den bekannten Wasserkastenprodukten ist jedoch gering, da die bekannten Mittel im Allgemeinen extrudierte Reinigungsmittelformkörper sind, die nur geringe Duftmittelmengen aufnehmen können, da sie ansonsten klebrig und nicht extrudierbar würden. Zudem erfüllen die bislang bekannten In-Tank-Produkte nicht die Verbraucherwünsche in Bezug auf die Transparenz.

Zur Anwendung im Wasserkasten ist bislang kein Mittel bekannt, das hohe Parfumkonzentrationen aufnehmen kann, hohe Standzeiten im Wasser des Wasserkastens erreicht, sich dennoch auflöst und transparent ist.

Die Aufgabe der vorliegenden Erfindung besteht darin, ein Sanitärmittel für den Wasserkasten von Toiletten anzugeben, das den Toilettenbereich über eine längere Zeitdauer beduftet, das sich nach und nach auflöst und transparent ist.

Diese durch wird durch eine Verwendung nach Anspruch 1 oder ein Verfahren nach Ansprunch 8 gelöst.

Erfindungsgemäß wird eine Kombination von wenigstens zwei Gelbildnern (Gelbildner 1 und Gelbildner 2) in dem Mittel eingesetzt.

Der eine Gelbildner (Gelbildner 1) dient zur Gelbildung mit den hydrophoben Duftstoffen und ist aus der Gruppe der Polyamidharze oder der Olefinhomopolymere und Copolymere von zwei und mehr Olefinen ausgewählt.

Als Gelbildner aus der Gruppe der Polyamidharze wird vorzugsweise ein Ester-terminiertes Polyamide (ETPA), ein Ester-terminiertes, auf dimerer Säure basierendes Polyamidharz (ETDABP), ein Amid-terminiertes Polyamide (ATPA) oder ein Polyalkylenoxy-Polyamide (PAOPA) eingesetzt. Diese Polyamidharze können mit hydrophoben Flüssigkeiten wie Parfümölen und Duftstoffen transparente klare Gele, die in Wasser schwer- oder unlöslich sind, bilden.

Als in Wasser schwer- bzw. unlöslicher Gelbildner 1 aus der Klasse der Ester-terminierten Polyamide (ETPA) kann eine Zusammensetzung der Formel 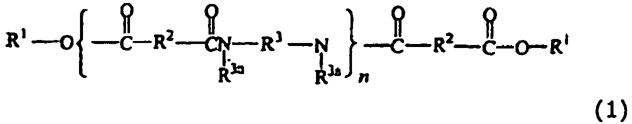 eingesetzt werden, wobei n eine Zahl an Wiederholungseinheiten bezeichnet, so dass Estergruppen von 10 % bis 50 % der Gesamtzahl der Ester- und Amidgruppen ausmachen; R¹ bei jedem Auftreten unabhängig ausgewählt ist aus einer Alkyl- oder Alkenylgruppe, welche wenigstens 4 Kohlenstoffatome enthalten; R² bei jedem Auftreten unabhängig ausgewählt ist aus einer C₄₋₄₂-Kohlenwasserstoffgruppe mit der Vorgabe, dass wenigstens 50 % der R²-Gruppen 30 - 42 Kohlenstoffatome aufweisen; R³ bei jedem Auftreten unabhängig ausgewählt ist aus einer organischen Gruppe, die wenigstens zwei Kohlenstoffatome zusätzlich zu Wasserstoffatomen enthält und optional ein oder mehrere Sauerstoff- und Stickstoffatome enthält; und R^{3a} bei jedem Auftreten unabhängig ausgewählt ist aus Wasserstoff, C₁₋₁₀-Alkyl und einer direkten Bindung an R³ oder einen weiteren R^{3a}, so dass das N-Atom, an welches R³ und R^{3a} beide angebunden sind, Teil einer heterocyclischen Struktur ist, die zum Teil durch R^{3a}-N-R³ definiert ist, so dass wenigstens 50 % der R^{3a}-Gruppen Wasserstoff sind.

Solche Verbindungenn sind in der EP 0 939782 B1 der Firma Arizona Chemical Co. beschrieben.

Als Ester-terminiertes, auf dimerer Säure basiertes Polyamidharz (ETDABP) kann eine Verbindung der folgenden Formel (2) ausgewählt werden: 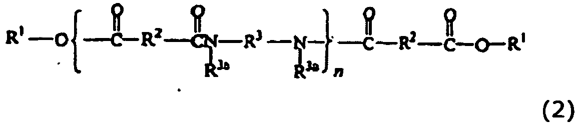 wobei n eine Anzahl von Wiederholungseinheiten bezeichnet, so dass Estergruppen von 10 % bis 50 % der Gesamtheit der Ester- und Amidgruppen ausmachen; R¹ bei jedem Auftreten unabhängig ausgewählt ist aus Kohlenwasserstoffgruppen; R² bei jedem Auftreten unabhängig ausgewählt ist aus einer C₂₋₄₂-Kohlenwasserstoffgruppe mit der Maßgabe, dass wenigstens 10 % der R²-Gruppe 30 - 42 Kohlenstoffatome aufweisen; R³ bei jedem Auftreten unabhängig ausgewählt ist aus einer organischen Gruppe, die wenigstens zwei Kohlenstoffatome zusätzlich zu Wasserstoffatomen enthält, und optional enthaltend ein oder mehrere Sauerstoff- und Stickstoffatome; und R³ bei jedem Auftreten unabhängig ausgewählt ist aus Wasserstoff, C₁₋₁₀-Alkyl und einer direkten Bindung an R³ oder ein weiteres R^{3a}, so dass das N-Atom, an das R³ und R^{3a} beide angebunden sind, Teil einer heterocyclischen Struktur ist, die teilweise durch R^{3a}-N-R³ definiert ist.

Solche Verbindungen sind in der EP 1 027 032 B1 der Firma Arizona Chemical Co. beschrieben.

Weiterhin können als Gelbildner 1 auch Olefinhomopolymere und Copolymere von zwei und mehr Olefinen eingesetzt werden. Zu diesen Verbindungen zählen z.B. die Polybutadienkautschuke, die StyrolButadien-Block- u. -copolymere sowie die Polyisopropene. Auch einsetzbar sind "random (block) polymers", die hergestellt werden durch 1,3-Addition von Butadien oder Isopren an Styrol oder alpha-Methyl Styrol, die Homopolymere oder Copolymere des Ethylens und Propylens, wie der Ethylen-Propylendien-Terpolymere, der natürliche Kautschuk und Norbornen-Polymere wie Polydicyclopentadien. Die Verbindungen aus der Gruppe der Olefinhomopolymere und Copolymere können auch teilhydriert sein.

Bei den Gelbildnern 1 aus der Gruppe der Polystyrolderivate handelt es sich vorzugsweise um in Mineralöl lösbare, quervernetzte Polystyrolderivate, insbesondere um Alkylenstyrol-Copolymere, wie beispielsweise den hydrierten Butylen/Ethylen/Styrol-Copolymeren und den hydrierten Ethylen/Propylen/Styrol-Copolymeren, die beispielsweise in gelöster Form von der Firma Penreco unter dem Handelsnamen Versagel M750 oder Versagel M1600 erhältlich sind. Diese Polymere selbst sind bei der Firma Shell als Kraton-Typen erhältlich.

Im Sinne der vorliegenden Erfindung sind die mit dem Gelbildner 1 und den Duftstoffen gelierten Gele dann mit Wasser schwer- oder unlöslich oder nicht vollständig dispergierbar, wenn ca. 0,5 Gramm des Mittels unter Zugabe von ca. 30ml Leitungswasser bei Raumtemperatur wenigstens 24 Stunden stehen gelassen wird und anschließend danach nach Schütteln des Gefäßes die überstehende Flüssigkeit durch Abdekantieren entfernt wird, wobei dieses Prozedere wenigstens 5 mal innerhalb von 8 Tagen wiederholt wird ohne dass eine vollständige Auflösung/vollständige Dispergierung des Mittels zu beobachten ist. Ist das Mittel nach 43 Tagen zudem wenigstens zu 10% gelöst, kann es im Sinne der Erfindung als Mittel eingesetzt werden. Mit anderen Worten sind solche Systeme ungeeignet, die schon nach wenigen Schütteltests vollständig gelöst sind und geeignet sind solche Mittel, die nach einem längeren Schüttelzyklus nach und nach in Lösung gehen. Im nachfolgend beschriebenen Ausführungsbeispiel V3 in Tabelle 1 ist das für den Spülkasten einer realen Toilette nach ca. 450 Spülungen bei einem Start-Gewicht von 13, 4 gr. der Fall.

Der zweite Gelbildner (Gelbildner 2) ist wasserlöslich oder wasserdispergierbar. Durch ihn kann nun die gewünschte Wasserlöslichkeit des Mittels und die gewünschten Spülzahlen eingestellt werden.

Der zweite Gelbildner wird aus der Klasse der Tenside, der wasserlöslichen oder wasserdispergierbaren Polyamidharze oder der gelbildenden natürlichen oder synthetischen Polymere ausgewählt.

Als Tenside werden vorzugsweise gelbildende anionische oder nichtionische Tenside eingesetzt, wobei sich Mittel mit Tensiden als Gelbildner 2 durch eine zusätzliche Reinigungswirkung auszeichnen.

Wesentlich ist, dass diese Tenside ebenfalls Gelbildner sind, da die Mittel ansonsten eintrüben und die gewünschte Transparenz verlieren würden.

Die anionischen Tenside erfordern wenigstens eine wasserlöslich machende anionische Gruppe wie z. B. eine Carboxylat-, Phosphat-, Sulfat-, Phosphonat-, Sulfonatgruppe und wenigstens eine lipophile Alkyl- und/oder Arylgruppe mit 8 bis 30 C-Atomen. Zusätzlich können weitere Gruppen wie beispielsweise Glykol-oder Polyglykolether-Gruppen, Ester-, Ether-und Amidgruppen, Hydroxylgruppen, jeweils in Form der Natrium-, Kalium-, Calcium-, Magnesium-, Zink-und Ammonium sowie der Mono-, Di-und Trialkanolammoniumsalze mit 2 bis 4 C-Atomen in der Alkanolgruppe im Molekül vorhanden sein.

Insbesondere können als gelbildende anionische Tenside die folgenden Verbindungen eingesetzt werden:
Acylisethionate mit 8 bis 30 C-Atomen in der Acylgruppe, Acylsarcoside mit 8 bis 30 C-Atomen in der Acylgruppe, Acyltauride mit 8 bis 30 C-Atomen in der Acylgruppe, Alkansulfonate (lineare) mit 8 bis 30 C-Atomen, Alkyl-, Aryl- und/oder Alkenyletherphosphate mit einen aliphatischen Kohlenwasserstoffrest mit 8 bis 30 Kohlenstoffatomen, Alkylpolyglykolethersulfate mit bevorzugt lineare Alkylgruppen, mit 8 bis 30 C-Atomen, Alkylsulfate, Alpha-Sulfofettsäuremethylester von Fettsäuren mit 8 bis 30 C-Atomen, Amid(o)ethercarbonsäuren, Eiweissfettsäurekondensate (Lamepon® -Typen, Amisoft® -Typen), Ethercarbonsäuren mit einer lineare Alkylgruppe mit 8 bis 30 C-Atomen, Fettsäurealkylenglykolester, Hydroxysulfonate, lineare Alpha-Olefinsulfonate mit 8 bis 30 C-Atomen, Monoglycerid(ether)sulfate, sulfatierte Hydroxyalkyl-/arylpolyethylen-und/oder Hydroxyalkylenarylenpropylenglykolether, Sulfobernsteinsäuremonoalkyl-/ arylpolyoxyethylester mit 8 bis 30 C-Atomen in der Alkyl-/Arylgruppe und 1 bis 6 Oxyethyl(propyl)gruppen, Sulfobernsteinsäuremono- und - dialky-/arylester mit 8 bis 30 C-Atomen in derAlkyl-/Arylgruppe und Sulfonate ungesättigter Fettsäuren mit 8 bis 30 C-Atomen und 1 bis 6 Doppelbindungen.

Auch Laurylethersulfate, Fettalkoholpolyethylenglykolethersulfat, Laurylbenzolsulfonate, Alkylpolyglykoletherphosphate sind einsetzbar.

Durch den Einsatz der sauren Alkylpolyglykoletherphosphate lässt sich der pH-Wert des Mittels einstellen, so dass auch ein saures Mittel erhältlich ist und das Mittel somit auch als Entkalkungsmittel eingesetzt werden kann.

Diese sauren anionischen Tenside sind als Phosphorsäureester weniger empfindlich gegenüber Härtebildnem des Wassers wie Calcium- oder Magnesiumionen, besitzen ein gutes Kalkseifendispergiervermögen, sind sehr beständig gegenüber Alkalien und mit anionischen, amphoteren und nichtionischen Tensiden verträglich. Weiterhin sind sie korrosionsschützend, vollständig biologisch abbaubar und entsprechen der Detergenzienverordnung der EG Nr. 648/2004.

Die sauren Alkylpolyglykoletherphosphate sind beispielsweise unter dem Handelsnamen Phosfetal von Zschimmer & Schwarz, Lahnstein, Deutschland, Crafol von der Firma Cognis oder Naxonac von der Firma Nease Performance Chemicals erhältlich.

Durch die Verwendung von Alkylbenzolsulfonaten als zweitem Gelbildner kann ebenfalls eine zusätzliche Reinigungswirkung und zudem auch eine Schaumentwicklung erzielt werden.

In einer besonders bevorzugten Variante werden sowohl Alkylbenzolsulfonat als auch Fettalkohol-Polyethylenglycolethersulfat in dem Mittel als zweiter bzw. weiterer Gelbildner eingesetzt.

Auch können als Gelbildner natürliche oder synthetische Polymere wie Cellulosen, insbesondere Natrium-Carboxymethylcellulosen, Hydroxyethylcellulosen, Hydroxypropylcellulose oder auch Polysaccharide wie Agar-Agar, Gummi Arabicum, Johannisbrotkernmehl oder Stärke, Polyacrylate, Polysaccharide, Polyvinylalkohole oder Polyvinylpyrrolidon, Alginate, Diurethane, Gelatine oder Pectine.

Als wasserlösliche oder wasserdispergierende Polyamidharze (Gelbildner 2) können beispielsweise Blockcopolymere der Formel Kohlenwasserstoff-Polyether-Polyamid-Polyether-Kohlenwasserstoff verwendet werden, wobei Copolymere, bei denen der Polyamidblock die Formel 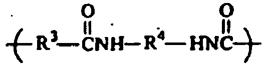 umfasst, worin R³ ein Kohlenwasserstoff ist und R⁴ aus Kohlenwasserstoffen und Polyethern ausgewählt ist, bevorzugt sind. Solche Verbindungen sind in der EP 1 358 248 B1 der Firma Arizona Chemical Co. beschrieben.

Weiterhin können als wasserlösliche Polyamidharze, beispielsweise Polyamid-6 oder Polyamid-4, die mit mittel- oder starkpolaren Flüssigkeiten vergelen und bei der Firma Arizona Chemical unter der Bezeichnung Silvaclear PE400V oder Silvaclear WF1500V erhältlich sind, eingesetzt werden. Das wasserlösliche Polyamidharz, das bei der Firma Arizona Chemical unter der Produktnummer X54-188-152 erhältlich ist, ist ebenfalls einsetzbar.

Der alleinige Einsatz des zweiten Gelbildners (ohne ersten Gelbildner) würde bei Lagerung des Mittels in Wasser dazu führen, dass sich das Mittel sofort oder nach wenigen Spülungen auflöst.

Insofern sieht die Erfindung die Kombination der beiden Gelbildner zusammen mit Parfüm vor.

Als Duftstoffe werden vorzugsweise Gemische von im Allgemeinen hydrophoben Parfümölen eingesetzt.

Der Duftstoffanteil kann in Mitteln mit Polyamidharzen bis zu 70 Gew.% betragen, so dass eine langanhaltende Beduftung der Toilette über das in dem Spülkasten in Wasser befindliche Mittel erreicht wird.

Die Konzentration des Gelbildners (1) sollte zwischen 5 und 80 Gew.%, vorzugsweise zwischen 8 und 60 Gew.% und besonders bevorzugt zwischen 15 und 50 Gew.% betragen, die des Gelbildners (2) sollte zwischen 5 und 80 Gew.%, vorzugsweise zwischen 8 und 60 Gew.% und besonders bevorzugt zwischen 15 und 50 Gew.%, und die Konzentration des Parfüms sollte insbesondere zwischen 1 und 40 Gew.%, vorzugsweise zwischen 3 und 30 Gew.% und besonders bevorzugt zwischen 4 und 15 Gew.%, betragen.

Das Konzentrationsverhältnis zwischen Gelbildner 1 : Gelbildner 2 sollte zwischen 95 und 5, vorzugsweise zwischen 90 und 10 und besonders bevorzugt zwischen 80 und 20 liegen.

Um die gewünschte Beduftung und das gewünschte Auflöseverhalten zu erreichen, sollen die beiden Gelbildner 1 und 2 in dem Mittel in einer Phase vorliegen.

Das erfindungsgemäße Mittel weist einerseits Spülzahlen von wenigstens 150 bei sterischer Hinderung wie beispielsweise wenigstens teilweiser Umhüllung mit einer anderen Masse von mehr als 200 bei permanenter Lagerung in kaltem Wasser auf, andererseits löst es sich jedoch nach und nach auf, so dass im Wasserkasten keine unerwünschten Rückstände verbleiben. Auf jeden Fall sollte sich das Mittel mit einer Masse von 50 g nach vorzugsweise bis zu 500 und maximal 800 Spülungen aufgelöst haben.

Zudem ist es gelungen, ein solches Mittel mit hohen Spülzahlen für den Wasserkasten auch als Mittel, das die Toilette über eine lange Zeitdauer beduften kann, bereitzustellen und gleichzeitig die gewünschte Transparenz des Mittels zu erhalten.

Ein Mittel ist im Sinne der vorliegenden Erfindung dann transparent, wenn es zum Zeitpunkt der ersten Ingebrauchnahme transparent ist. Ob das Mittel im Gebrauch im Wasserkasten nach und nach an Transparenz verliert ist unerheblich, da zu diesem Zeitpunkt das Mittel für den Verbraucher nicht mehr sichtbar ist.

Die Transparenz des Mittels wird dadurch bestimmt, ob noch Schriften mit abnehmender Größe durch verschiedene Schichtdicken des Mittels lesbar sind. Zur Feststellung der Transparenz des Mittels wurden die Ansätze in Aluminiumformen (Innenmaß: 5 cm oder 2,5 cm, Dicke x 2 cm, Breite x ca. 8 cm Höhe) gegossen, über Nacht abgekühlt und am nächsten Tag aus der Form gepresst.

Beurteilt wurde die Lesbarkeit einer Schrift von 3, 5 bzw. 7 Pixeln Höhe, die auf einem HP Laserjet 2100 erstellt wurde, wobei ein Mittel dann im Sinne der Erfindung transparent ist, wenn wenigstens eine 7 PixelSchicht durch eine 25 mm Schichtdicke lesbar ist, besonders gute Transparenz liegt vor, wenn die Lesbarkeit einer 5 bzw. 3 Pixelschicht durch einen 49 mm dicken Block noch lesbar ist.

Die erfindungsgemäßen Mittel zeichnen sich weiterhin auch durch eine genügende Härte bei Raumtemperatur aus. Die Mittel sind bei Raumtemperatur schnittfest und formstabil.

Die Erfindung betrifft auch ein Verfahren zur Beduftung von Toiletten. Die Beduftung erfolgt dadurch, dass das zuvor beschriebene Sanitärmittel in den Wasserkasten einer Toilette hineingegeben wird. Das Mittel löst sich in kaltem Wasser nach und nach auf und setzt kontinuierlich Duftstoffe frei, die die Toilette beduften.

Die Erfindung wird nachfolgend anhand von Ausführungsbeispielen näher beschrieben.

### 1. Erfindungsgemäße Ausführungsbeispiele:

Die nachfolgende Tabelle enthält die verschiedenen erfindungsgemäßen Ausführungsbeispiele.

**Tabelle 1**

| | | **V1** | **V2** | **V3** | Hersteller | |
|---|---|---|---|---|---|---|
| | **Material** | **[%] Einwaage** | **[%] Einwaage** | **[%] Einwaage** | | |
| | PE 400 V | 60 | 58 | | Arizona Chemical | Gelbildner 1 |
| | AF 1900 | 2 | 7 | | Arizona Chemical | Gelbildner 1 |
| | PA 1200 | | | 66 | Arizona Chemical | Gelbildner 1 |
| | Phosfetal 201 | 23 | | | Zschimmer &Schwarz | Gelbildner 2 |
| | Marlinat 242/90T | | 20 | 30 | Sasol | Gelbildner 2 |
| | Orange Fun | 15 | 15 | 4 | Givaudan | Parfüm |
| Einwaage Stein | | 31 gr | 30,6 gr | 13,4 gr | | |
| Transparenz durch eine 25 mm dicke Block; Schriftgröße 7 Pixel | | lesbar | lesbar | n.b. | | |
| Transparenz durch 49 mm breiten Block; 3pt Schriftgröße | | lesbar | nicht mehr lesbar | n.b. | | |
| Transparenz in Spülkasten nach 12 h | | wird weiß | wird weiß | bleibt transparent | | |
| Oberflächenspannung | | 54,4; 48,2; 45,9 | 56,7;50,1; 47,6 | n.b. | | |
| Schaum | | 80;75;50 | 90; 80; 75 | n.b. | | |
| pH-Wert; 20,6°C | | 3,78 | 4,79 | n.b. | | |
| Spülzahl | | ca 30 | ca 30 | > 450 | | |

Als Alkylpolyglykoletherphosphat wurde Phosfetal 205, erhältlich bei Zschimmer & Schwarz, eingesetzt. Der pH-Wert einer 1% Lösung beträgt 2. Die Alkylgruppe ist eine C14- C18-Gruppe, die "Polyglykolether"-Gruppe weist zwischen 1 und 3 Glykoleinheiten auf.

Marlinat 242/90T besteht aus C12-C14-Alkohol-Polyethylenglykol und Propylenether-(2 EO)-Sulfat, Glykoltriisopropanolammoniumsalz (Fa. Sasol)

### Bestimmung der Spülzahlen:

Die Spülzahlen wurden derart bestimmt, dass das Mittel in den Spülkasten eingeworfen oder in den Korb eingehängt wurde, wobei der Füllstand ca. 8 Liter Wasser bei einer Temperatur von 16 °C betrug.

Pro Spülung wurde vollständig entleert und dann wieder auf 8 Liter aufgefüllt.

Anschließend wurde die Anzahl der Spülungen ermittelt, die bis zur vollständigen Auflösung des Mittels erforderlich waren.

### Versuchsauswertung:

1) Es entstehen transparente, z. T. hochtransparente Massen.
2) Die Massen sind alle tensid- und parfümhaltig.
3) Es können sauer eingestellte Systeme hergestellt werden.
4) Es gibt Systeme (z. B. V3), die hohe Spülzahlen, ähnlich wie extrudierte Steine aufweisen und transparent bleiben!

### 2. Vergleichsversuche aus dem Stand der Technik

**Tabelle 2**

| | | |
|---|---|---|
| | DE 197 10 635 | WO 99 / 66017 |
| Gelbildner | 31,6 % Polyoxyethylen 10 | 13 % Alkylpolyglykolether 35 EO |
| Gelbildner | 19,2 % Polyoxyethylen 11 | 13 % Alkylpolyglykolehter 30 EO |
| Gelbildner | 5,3 % Na-Stearat | |
| Duftstoff | 32,60% | 6,50 % |
| sonstiges | 4,7 % Wasser | 6 % Marlinat 242/90T |
| | 4,7 % Propylenglycol | 0,003 % Farbstoff |
| | 0,09 % Farbstoff | 48 % Wasser |
| | 1,8 % DBS, Na-Salz | 1,3 % Polyethylenglykol 6000 |
| | | 12 % Glycerin (86,5-prozentig) |
| Transparenz | nicht lesbar durch 5er Schrift bei 49 mm Schichtdicke (wird mit der Zeit trüb) | Sehr gut, bleibend |
| Wasserlöslichkeit innerhalb von weniger als 15 Spülzyklen im Wasserkasten | vollständig | vollständig |
| Duftstoffaufnahme | bis zu 35% | ∼ 6,5 % |
| Aggregatzustand | fest, nicht formstabil | hochviskos |
| pH-Wert (1% Wasser dest.) | ca. 7 | ca. 7 |
| Schaumzahl 1% Lösung | + 60 mL | + 80 mL |
| Oberflächen-spannung 100 ms (1% Lösung) | 47 mN/m | 66 mN/m |
| Gelbildner | Stearat/Nichtionische Tenside | Nichtionische Tenside |

### Auswertung:

Die bisherigen Systeme sind zwar mit hohen Duftstoffkonzentrationen belastbar und auch transparent, aber weder fest genug, noch ausreichend spülfest, um als Mittel im Spülkasten eingesetzt zu werden. Zudem können sie nicht sauer eingestellt werden.

### 3. Tabelle verschiedener erfindungsgemäßer Rezepturen

Die Tabelle 3 zeigt verschiedene Mischungen der Gelbildner 1 und 2 mit Parfümzusatz; das Löseverhalten zeigen die Tabellen 4 und 5.

**Tabelle 3**

| | **Versuchsbezelchnung** | | | | | | | | | | |
|---|---|---|---|---|---|---|---|---|---|---|---|
| | **AF TS 10** | **AF TS 40** | **A200 TS 10** | **A200 TS 40** | **PA TS 10** | **PA TS 40** | **Imb 5** | **Imb 40** | **Teqo 5** | **Teqo 40** | **Gelatine 40** |
| **Rohstoff** | Einwaage [%] | | | | | | | | | | |
| **Polyamid AF 1900** | 87,2 | 67,0 | | | | | 90,7 | 68,5 | 87,9 | 68,7 | 68,9 |
| **Polyamid A 200** | | | 85,4 | 66,8 | | | | | | | |
| **Polyamid PA 1200** | | | | | 86,4 | 66,3 | | | | | |
| **Marlinat 242/90T** | 8,9 | 28,0 | 10,3 | 28,6 | 9,3 | 29,3 | | | | | |
| **Imbentin AG** | | | | | | | 4,7 | 27,8 | | | |
| **Tego Carbomer 134** | | | | | | | | | 13,1 | 27,8 | |
| **Gelatine** | | | | | | | | | | | 27,5 |
| **Parfüm Orange Fun** | 3,9 | 5,0 | 4,3 | 4,6 | 4,3 | 4,4 | 4,6 | 3,7 | 4 | 3,5 | 3,6 |
| **PA/Parfüm** | 4,47% | 7,46% | 5,04% | 6,89% | 4,9846 | 6,64% | 5,07% | 5,40% | 4,83% | 5,09% | 5,22% |
| G2/G1 | 10,21 | 41,79 | 12,06 | 42,81 | 10,76 | 44,19 | 5,18 | 40,58 | 15,80 | 40,47 | 39,91 |
| | | 100,00 | 100,00 | 100,00 | 100,00 | 100,00 | 100,00 | 100,00 | 100,00 | 100,00 | 100,00 |

| | |
|---|---|
| Imbentin AG: | Imbentin AG/168/110 Fa. Kolb Chemie |
| Gelatine: | Fa. Merck |
| Tego Carbomer: | Fa. Goldschmidt AG |
| G2, G1: | Gelbildner 2 und Gelbildner 1 |

### 4. Löslichkeitsverhalten des erfindungsgemäßen Versuchs aus Tabelle 3

Die halbquantitativen Schüttel- und Löseversuche mit verschiedenen Gelbildner 1/Gelbildner 2-Kombinationen sind in der nachfolgenden Tabelle 4 dargestellt.

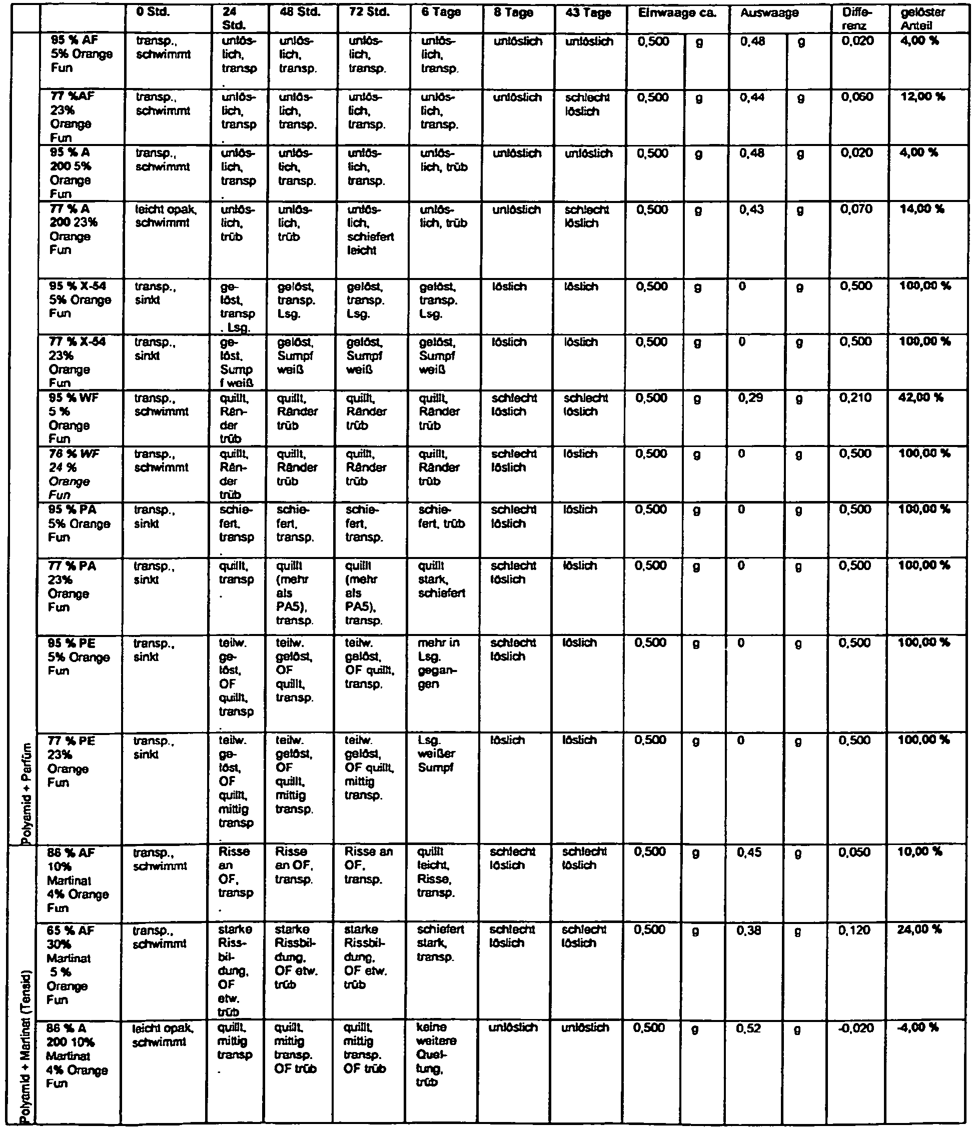

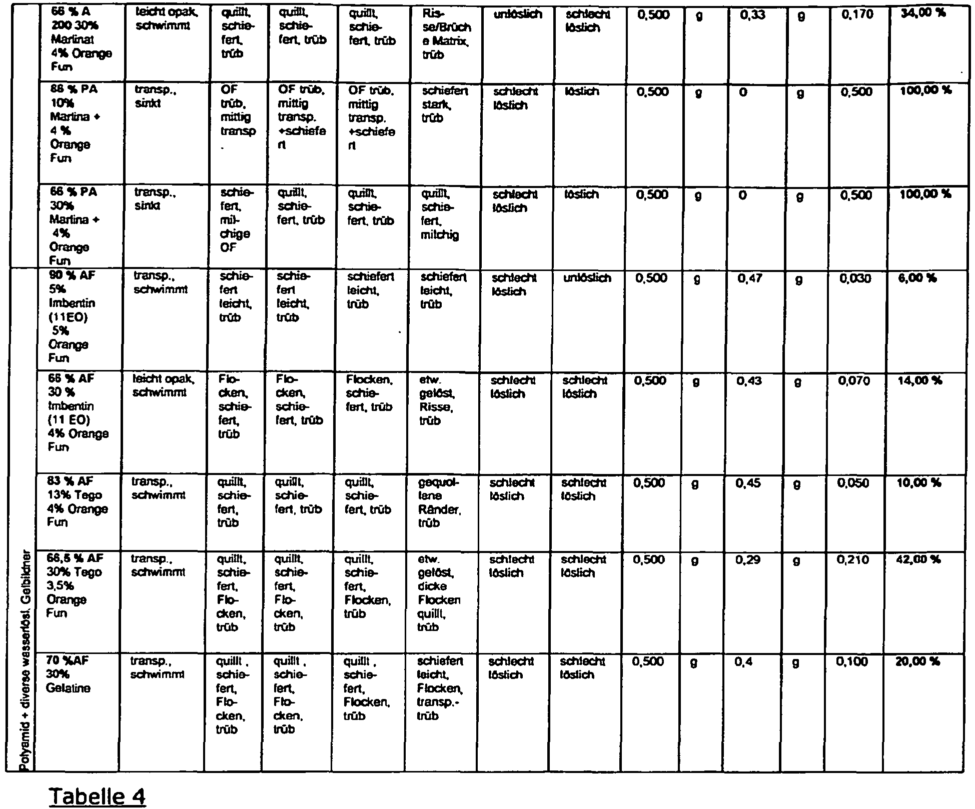

Die Tabelle zeigt den Stand der Schüttelversuche nach 8 Tagen und nach 43 Tagen; nach 43 Tagen wurde quantitativ ausgewertet; dazu wurden die überstehenden Lösungen ausgeschüttet und anschließend der Rückstand erst 24 h bei 40°C getrocknet und danach für ca. 50 h bei Raumtemperatur stehen gelassen. Die Einwaagen lagen bei ca. 0,5 gr (+/- 5 % Wägefehler)

Die Löslichkeitstests wurden in 30 g Leitungswasser mit 0,5 g Masse in verschließbaren Gefäßen bei Zimmertemperatur (stehen lassen) durchgeführt.

Die Versuche simulieren eine Lagerung und Spülung im WC, wobei allerdings nur maximal einmal am Tag gespült wird. Spülen bedeutet, dass die überstehende Flüssigkeit zuerst geschüttelt wird (simuliert die Verwirbelung im Wasserkasten) und anschließend abdekantiert. Danach wird wieder mit frischem Leitungswasser aufgenommen.
AF = Sylvaclear AF 1900
A 200 = Sylvaclear A 200
X-54 = Sylvaclear X-54-188-152
WF = Sylvaclear WF 1500
PA = Sylvaclear PA 1200
PE = Sylvaclear PE 400 V
Orange Fun = Parfüm
Marlinat = Marlinat 242/90T
Imbentin = Imbentin AG/168S/110
Tego = Tego Carbomer 134 Polyacrylat
OF = Oberfläche

## Patentansprüche

1. Verwendung eines stückförmigen Sanitärmittels zur Anwendung im Wasserkasten einer Toilette, welches Mittel wenigstens zwei verschiedene Gelbildner (1, 2) und Duftstoffe umfasst,
wobei der eine Gelbildner (Gelbildner 1) aus der Gruppe der Polyamidharze oder der Olefinhomopolymere und Copolymere von zwei und mehr Olefinen ausgewählt wird und mit hydrophoben Flüssigkeiten in Wasser unlösliche Gele bildet und der Anteil an Gelbildner 1 in dem Mittel wenigstens 30 Gew.% beträgt,
und der andere Gelbildner (Gelbildner 2) ein Tensid, ein wasserlösliches oder wasserdispergierbares Polyamidharz oder ein natürliches oder synthetisches gelbildendes Polymer ist und wasserlöslich oder wasserdispergierbar ist
und wobei das Mittel transparent ist, sich nach und nach abspült und Duftstoffe freisetzt und Gelbildner 1 und 2 in einer Phase vorliegen.

2. Verwendung nach Anspruch 1, **dadurch gekennzeichnet, dass** das Polyamidharz aus der Gruppe der Ester-terminierten Polyamide (ETPA), der Ester-terminierten, auf dimerer Säure basierendes Polyamidharz (ETDABP), der Amid-terminierten Polyamide (ATPA) und der Polyalkylenoxy-Polyamide (PAOPA) ausgewählt wird und das Olefinhomopolymer oder Copolymer von zwei oder mehr Olefinen aus der Gruppe der Polybutadienkautschuke, der Styrol-Butadien-Blocku. -copolymere sowie der Polyisopropene ausgewählt wird.

3. Verwendung nach einem der vorangegangenen Ansprüche, **dadurch gekennzeichnet, dass** das Tensid ein anionisches, nichtionisches oder amphoteres Tensid ist, wobei das anionische Tensid vorzugsweise aus der Gruppe der Ethersulfate, der Sulfonate, Phosphat ausgewählt wird und insbesondere ein Alkylbenzolsulfonat, ein Fettalkoholpolyethylenglyolehtersulfat, ein Alkylethersulfat oder ein Alkylpolyglykoletherphosphat ist.

4. Verwendung nach einem der vorangegangenen Ansprüche, **dadurch gekennzeichnet, dass** der Duftstoffanteil weniger als 70 % und zwischen 1 und 40 Gew.%, vorzugsweise zwischen 3 und 30 Gew.% und besonders bevorzugt zwischen 4 und 15 Gew.% beträgt.

5. Verwendung nach einem der vorangegangenen Ansprüche, **dadurch gekennzeichnet, dass** der Anteil an Gelbildner 1 in dem Mittel wenigstens 40 Gew.% und besonders bevorzugt wenigstens 50 Gew.%, beträgt.

6. Verwendung nach einem der vorangegangenen Ansprüche, **dadurch gekennzeichnet, dass** der Anteil des Gelbildners 2 in dem Mittel zwischen 5 und 80 Gew.%, vorzugsweise zwischen 8 und 60 Gew.% und besonders bevorzugt zwischen 15 und 50 Gew.% beträgt.

7. Verwendung nach einem der vorangegangenen Ansprüche, **dadurch gekennzeichnet, dass** die Spülzahlen des in einem Wasserkasten befindlichen Mittels bei 16 °C wenigstens 150, vorzugsweise wenigstens 200, betragen.

8. Verfahren zur Beduftung von Toiletten, **dadurch gekennzeichnet, dass** ein stückförmiges transparentes Sanitärmittel, das wenigstens zwei verschiedene Gelbildner (1, 2) und Duftstoffe umfasst, wobei der eine Gelbildner (Gelbildner 1) aus der Gruppe der Polyamidharze oder der Olefinhomopolymere und Copolymere von zwei und mehr Olefinen ausgewählt wird und mit hydrophoben Flüssigkeiten Gele bildet, die Gele in Wasser unlöslich sind und der Anteil an Gelbildner 1 in dem Mittel wenigstens 30 Gew.% beträgt, und der andere Gelbildner (Gelbildner 2) ein Tensid, ein wasserlösliches oder wasserdispergierbares Polyamidharz oder ein natürliches oder synthetisches gelbildendes Polymer ist und wasserlöslich oder wasserdispergierbar ist und Gelbildner 1 und 2 in einer Phase vorliegen, in den Wasserkasten einer Toilette hineingegeben wird, wodurch es sich nach und nach abspült und Duftstoffe freisetzt.

## Claims

1. The use of a sanitary product in piece form for use in the cistern of a toiled, which product comprises at least two different gel formers (1, 2) and fragrances,
wherein one gel former (gel former 1) is selected from the group of the Polyamide resins or the olefin homopolymers and copolymers of two and more olefins and, with hydrophobic liquids, forms water-insoluble gels and the fraction of gel former 1 in the product is at least 30% by weight, and the other gel former (gel former 2) is a surfactant, a water-soluble or water-dispersible polyamide resin or a natural or synthetic gel-forming polymer and is water-soluble or water-dispersible
and wherein the product is transparent, gradually flushes away and releases fragrances, and gel formers 1 and 2 are in one phase.

2. The use as claimed in claim 1, **characterized in that** the Polyamide resin is selected from the group of the ester-terminated polyamides (ETPA), the ester-terminated, dimeric-acid-based polyamide resins (ETDABP), the amide-terminated polyamide (ATPA) and the polyalkyleneoxy-polyamides (PAOPA), and the olefin homopolymer or copolymer of two or more olefins is selected from the group of the polybutadiene rubbers, the styrene-butadiene block polymers and copolymers, and the polyisopropenes.

3. The use as claimed in any one of the preceding claims, **characterized in that** the surfactant is an anionic, nonionic or amphoteric surfactant, where the anionic surfactant is preferably selected from the group of the ether sulfates, the sulfonates, phosphates and is in particular an alkylbenzenesulfonate, a fatty alcohol polyethylene glycol ether sulfate, an alkyl ether sulfate or an alkyl polyglycol ether phosphate.

4. The use as claimed in any one of the preceding claims, **characterized in that** the fragrance fraction is less than 70% and between 1 and 40% by weight, preferably between 3 and 30% by weight and particularly preferably between 4 and 15% by weight.

5. The use as claimed in any one of the preceding claims, **characterized in that** the fraction of gel former 1 in the product is at least 40% by weight and particularly preferably at least 50% by weight.

6. The use as claimed in any one of the preceding claims, **characterized in that** the fraction of gel former 2 in the product is between 5 and 80% by weight, preferably between 8 and 60% by weight and particularly preferably between 15 and 50% by weight.

7. The use as claimed in any one of the preceding claims, **characterized in that** the flush numbers of the product located in a cistern at 16°C are at least 150, preferably at least 200.

8. A method for scenting toilets, **characterised in that** a transparent sanitary product in piece form which comprises at least two different gel formers (1, 2) and fragrances, where one gel former (gel former 1) is selected from the group of the Polyamide resins or the olefin homopolymers and copolymers of two and more olefines and forms gels with hydrophobic liquids, which gels are insoluble in water, and the fraction of gel former 1 in the product is at least 30% by weight and the other gel former (gel former 2) is a surfactant, a water-soluble or water-dispersible polyamide resin or a natural or synthetic gel-forming polymer and is water-soluble or water-dispersible, and gel formers 1 and 2 are in one phase, is placed into the cistern of a toilet, whereupon it gradually flushes away and releases fragrances.

## Revendications

1. Utilisation d'un produit sanitaire en forme de pièce pour l'application dans le réservoir d'eau de toilettes, ledit produit comprenant au moins deux gélifiants (1, 2) différents et des parfums, un des gélifiants (gélifiant 1) étant choisi dans le groupe des résines de polyamide ou des homopolymères oléfiniques et des copolymères de deux oléfines et plus, et formant des gels insolubles dans l'eau avec des liquides hydrophobes, et la proportion de gélifiant 1 dans le produit étant d'au moins 30 % en poids,
et l'autre gélifiant (gélifiant 2) étant un tensioactif, une résine de polyamide soluble dans l'eau ou dispersible dans l'eau, ou un polymère gélifiant naturel ou synthétique, et étant soluble dans l'eau ou dispersible dans l'eau,
et le produit étant transparent, se rinçant petit à petit et libérant des parfums, et les gélifiants 1 et 2 se présentant dans une phase.

2. Utilisation selon la revendication 1, **caractérisée en ce que** la résine de polyamide est choisie dans le groupe des polyamides à terminaison ester (ETPA), des résines de polyamide à base d'acide dimère à terminaison ester (ETDABP), des polyamides à terminaison amide (ATPA) et des polyalkylène-oxy-polyamides (PAOPA), et l'homopolymère oléfinique ou le copolymère de deux oléfines ou plus étant choisi dans le groupe des caoutchoucs de polybutadiéne, des copolymères séquence de styrène-butadiène, ainsi que des polyisoprènes.

3. Utilisation selon l'une quelconque des revendications précédentes, **caractérisée en ce que** le tensioactif est un tensioactif anionique, non ionique ou amphotère, le tensioactif anionique étant de préférence choisi dans le groupe des éther-sulfates, des sulfonates, des phosphates, et étant notamment une sulfonate d'alkylbenzène, un éther-sulfate de polyéthylène glycol-alcool gras, un éther-sulfate d'alkyle ou un éther-phosphate d'alkylpolyglycol.

4. Utilisation selon l'une quelconque des revendications précédentes, **caractérisée en ce que** la proportion de parfums est de moins de 70 % et comprise entre 1 et 40 % en poids, de préférence entre 3 et 30 % en poids, et de manière particulièrement préférée entre 4 et 15 % en poids.

5. Utilisation selon l'une quelconque des revendications précédentes, **caractérisée en ce que** la proportion du gélifiant 1 dans le produit est d'au moins 40 % en ponds et de manière particulièrement préférée d'au moins 50 % en poids.

6. Utilisation selon l'une quelconque des revendications précédentes, **caractérisée en ce que** la proportion du gélifiant 2 dans le produit est comprise entre 5 et 80 % en poids, de préférence entre 8 et 60 % en poids et de manière particulièrement préférée entre 15 et 50 % en poids.

7. Utilisation selon l'une quelconque des revendications précédentes, **caractérisés en ce que** le nombre de rinçages du produit se trouvant dans une réservoir d'eau à 16 °C est d'au moins 150, de préférence d'au moins 200.

8. Procédé de parfumage de toilettes, **caractérisé en ce qu'**un produit sanitaire transparent en forme de pièce, qui comprend au moins deux gélifiants (1, 2) différents et des parfums, un des gélifiants (gélifiant 1) étant choisi dans le groupe des résines de polyamide ou des homopolymères oléfiniques et des copolymères de deux oléfines et plus, et formant des gels insolubles dans l'eau avec des liquides hydrophobes, et la proportion de gélifiant 1 dans le produit étant d'au moins 30 % en poids, et l'autre gélifiant (gélifiant 2) étant un tensioactif, une résine de polyamide soluble dans l'eau ou dispersible dans l'eau, ou un polymère gélifiant naturel ou synthétique, et étant soluble dans l'eau ou dispersible dans l'eau, et les gélifiants 1 et 2 se présentant dans une phase, est introduit dans le réservoir d'eau de toilettes, où il se rince petit à petit et libère des parfums.
